# EUROPEAN PATENT APPLICATION

(11) **EP 1 602 323 A1**
(43) Date of publication of application: **07.12.2005**
(21) Application number: 04468018.9
(22) Date of filing: 15.10.2004
(51) Int. Cl.: A61B 3/135, A61B 3/00

(54) **Slit lamp incorporating a white light emitting diode or a light emitting diode with adjustable colour**

(30) Priority: 04.06.2004 SI 200400158 P
(71) Applicant: Optotek d.o.o., 1000 Ljubljana (SI); Dioptrix, 31240 L'Union (FR)
(72) Inventor: Mocnik, Grisa, 1000 Ljubljana (SI); Zalar, Matjaz, 1294 Grosuplje (SI)
(74) Representative: Flak, Antonija

(57) **Abstract**

The light source 1 in the slit lamp (Figure 1) according to present invention is a white light emitting diode (LED) controlled by a control circuit 8. The patient's eye is illuminated by an illuminating slit produced as image 7 of the slit 3 by means of Koehler illumination technique.

This means that the phosphorescent surface of the LED is projected through the condenser optics 2 onto the rear objective 4 that makes an image of the slit 3 through a prism or a mirror 6 onto the eye. In this way an evenly illuminated image 7 of the slit 3 is formed on the inspected eye. The control circuit 8 ensures a correct ratio between the voltage across the LED and the current flowing through the LED and enables changing of the emitted light intensity. The colour of the light does not vary with the intensity of the light. In an embodiment of the invention, the light source of the slit lamp is a LED emitting simultaneously a red, a green, and a blue light. By adjusting the intensity of individual light components, optional colour of the slit image 7 can be produced on the eye. So, for coloured illumination of the eye, the colour filters, which must otherwise be inserted into a white illuminating beam, are not needed.

## Description

### Field of the invention

The invention relates in general to medical devices for eye diagnostics, in particular, to illuminating means for examining the eyes.

### Technical problem

The technical problem solved by the present invention is to design a light source in a diagnostic lamp for slit illumination to provide a uniform illumination across the entire slit surface. Besides, when regulating the intensity of the illumination, the light source shall not change its colour. In addition, the solution of the illumination shall ensure that the consumption of electric energy is small and that heating of the slit illuminator is as small as possible. The design of a light source should be such as to allow building-in into portable slit lamps.

### Background to the disclosure

Slit lamps are devices for eyes examination. A slit lamp produces an illuminating slit with adjustable width to enable the ophthalmologist to observe parts of the eye selectively. It is very important that the illumination is even across the entire surface of the slit and that the intensity of the illumination can be regulated. The ophthalmologist watches the illuminated part of the eye through a binocular stereomicroscope with an up to 40 times magnification. The change of colour of the illuminating light, which is needed in some examinations, can be achieved by different colour filters.

In known solutions of slit lamps, the slit is illuminated predominantly by a filament bulb, mostly by a halogen illuminant. Due to high consumption of electric energy, such illuminants warm themselves, and their transportability is limited, as well. Besides, they are sensitive and due to warming they have a short lifespan, even less than 100 hours. As the current through the filament controls the intensity of the illumination, the filament temperature is changing with the intensity, causing the colour of the light to vary, that can be very disturbing for the user of the slit lamp. For examination of eyes mostly a slightly bluish light is desired, that is produced by the filament at a high temperature, which means at high intensity of the light.

A uniform illumination of individual parts of the eye can be achieved by a Koehler illumination technique, using condenser optics to image the filament through a slit onto a rear objective, where an image of the filament appears. The rear objective images the light slit onto the eye. To protect the eye against heat injury that can be caused by the infrared light, an infrared filter is built-in after the slit.

In the patent US 4,699,482, the illumination using optical fibers is described. The illumination by means of modulators of light composed of chip components with liquid crystals is described in the patent US 5,943,118. By the solution from US 4,699,482 a sufficient evenness of illumination cannot be achieved, while in the solution according to US 5,943,118, a halogen bulb or a similar light source is used. According to patent US 6,409,346 the illumination of the slit is solved by several diodes or diode chips, which do not enable a satisfactory uniformity of the slit illumination.

Beside white light, colour light is also used in slit lamps, especially blue and green light, which is produced by inserting adequate filters into the illuminating light beam. The colour is determined by the type of filter or by several filters, which complicates the construction as well as the use of the device.

### Description of the invention

The essential feature of the slit lamp with a light emitting diode (LED) according to the invention is in that the control circuit 8 controls the light source 1, the light source being either a white LED or a LED with adjustable colour that lights through condenser optics 2, a slit 3, and a rear objective 4, and through a prism or a mirror 6 onto the eye, where a slit image 7 is produced. The control circuit 8 enables regulation of the intensity of the light emitted by the LED and ensures a correct ratio between the voltage across the LED and the current flowing through the LED.

A slit lamp with light emitting diode according to the invention is explained in detail by means of figures showing:
- Figure 1 -: a block diagram of the slit lamp with a white LED

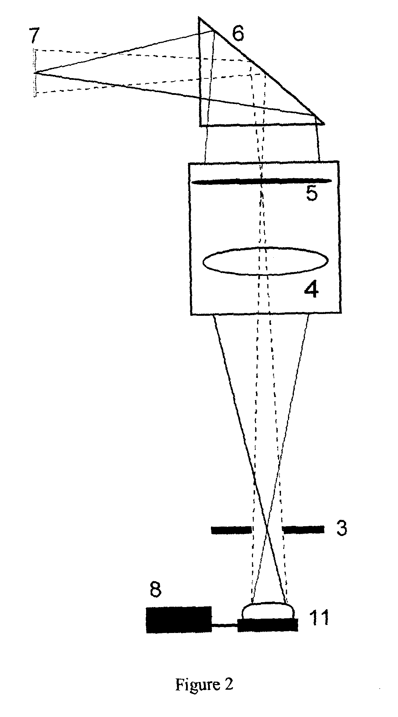

- Figure 2 -: a block diagram of the slit lamp incorporating a white LED with integrated condenser optics

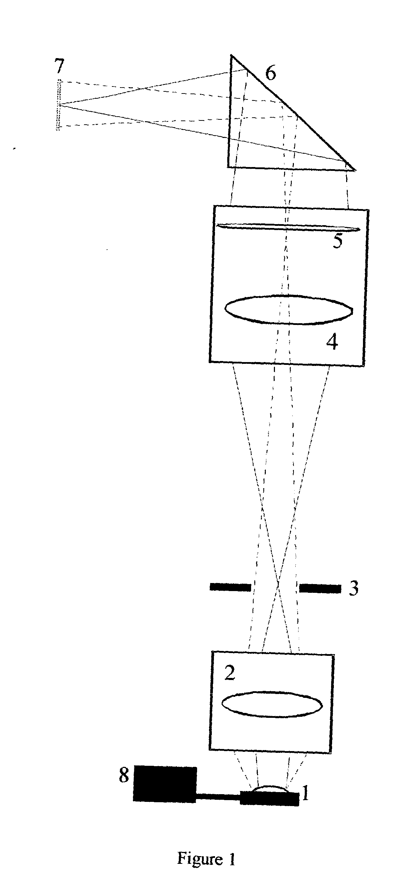

The light source 1 in the slit lamp according to present invention (Figure 1) is a white LED that is controlled by the control circuit 8. The patient's eye is illumined by a white illuminating slit that is produced as an image 7 of the slit 3 by means of Koehler illumination technique. This means that the phosphorescent surface of the LED is projected through the condenser optics 2 onto the rear objective 4 that makes an image of the slit 3 through a prism or a mirror 6 onto the eye. In this way an evenly illuminated image 7 of the slit 3 is formed on the inspected eye.

A white LED is a small impedance load therefore small changes of voltage across the LED can cause big changes of the current flowing through it. Consequently, the current must be regulated precisely across the whole range of the operation of the LED. In the slit lamp according to the invention a correct ratio between the voltage across the LED and the current flowing through the LED is ensured by means of the control circuit 8, which also takes care for the desired changing of the light intensity. The intensity of the light emitted by the phosphorescent surface of the LED is regulated by means of the pulse width of the current that has a fixed frequency and amplitude, and can be adjusted in the range from 0 to 100 % of the maximum intensity. The colour of the light does not vary with the intensity of the light.

In the second embodiment of the invention, the light source of the slit lamp is a white LED 11 having the condenser optics integrated in the LED 11.

In the third embodiment of the invention, the light source of the slit lamp is a LED emitting simultaneously a red, a green, and a blue light. By adjusting the intensity of individual light components, optional colour of the slit image 7 can be produced on the eye. So, for coloured illumination of the eye, the colour filters, which must otherwise be inserted into a white illuminating beam, are not needed.

With the slit lamp according to the invention incorporating a white LED, the examination of the eye is improved and facilitated as the illuminating slit on the eye is white and has even distribution of light across its surface, and because the colour of the light beam does not change with the light intensity. The advantage of the slit lamp according to the invention is also in a prolonged time between maintenance procedures as the lifespan of a LED is much longer than the lifespan of a halogen bulb. Besides, due to lower energy consumption the slit lamp according to the invention is suited for portable construction.

By applying light emitting diodes as light sources in slit lamps, the time between maintenance procedures can be extended considerably, even up to 1000 times. An important consideration is also several times lower energy consumption as compared to halogen light sources. Smaller energy consumption also means that the heating, which is generally not wanted, is smaller too.

A LED light source in the slit lamp has some other advantages as compared to filament bulbs or halogen illuminants. No special filters are necessary for infrared radiation as in the case of a hot filament bulb. The LEDs are cheaper than special halogen illuminants that must be applied in slit lamps, therefore the devices with LEDs are cheaper and require lower maintenance costs. As the energy consumption of the LEDs is low, the portable slit lamps can be used for longer periods of time without intermediate charging, which is an important gain for the fieldwork.

## Claims

1. A slit lamp with light emitting diode **characterized in that** the light source (1) in the slit lamp is a white light emitting diode (LED) and that the white light of the LED is conveyed through a condenser optics (2), a slit (3), a rear objective (4), and through a prism or a mirror (6) onto the eye, where a slit image (7) is produced.

2. A slit lamp according to claim 1, wherein the light source (1) is a white LED (11) with integrated condenser optics.

3. A slit lamp according to claim 1, wherein the light source (1) is a LED emitting simultaneously a red, a green, and a blue light and wherein the intensity of said individual colour components is controllable, so that it is possible to adjust optional colour of the illumination of the slit image (7) on the eye.

4. A slit lamp according to any of claims 1 to 3, wherein the slit lamp is designed as either a stationary construction or a mobile construction.

5. Use of a white LED for illuminating the slit in a slit lamp, which is designed as either a stationary construction or a mobile construction.

6. Use of a LED with controllable colour of emitted light for illuminating the slit in a slit lamp, which is designed as either a stationary construction or a mobile construction.
